# EUROPÄISCHE PATENTSCHRIFT

(11) **EP 2 942 614 B1**
(45) Veröffentlichungstag und Bekanntmachung des Hinweises auf die Patenterteilung: **20.01.2021**
(21) Anmeldenummer: 15157737.6
(22) Anmeldetag: 05.03.2015
(51) Int. Cl.: G01N 21/31, G01N 33/483, G01N 21/17, G01N 21/80, G01N 21/85

(54) **VORRICHTUNG UND VORRICHTUNGS-STEUERUNGSVERFAHREN ZUR QUANTITATIVEN KONZENTRATIONSBESTIMMUNG AUSGEWÄHLTER AUS EINEM PATIENTENKÖRPER AUSGEFILTERTER SUBSTANZEN IN EINER FLÜSSIGKEIT**
DEVICE AND DEVICE CONTROL METHOD FOR QUANTITATIVELY DETERMINING THE CONCENTRATION OF SELECTED SUBSTANCES IN A LIQUID FILTERED OUT OF A PATIENT'S BODY
DISPOSITIF ET PROCÉDÉ DE COMMANDE DE DISPOSITIF DESTINÉ À LA DÉTERMINATION DE CONCENTRATION QUANTITATIVE DE SUBSTANCES SÉLECTIONNÉES DANS UN LIQUIDE EXTRAITES PAR FILTRATION D'UN CORPS D'UN PATIENT

(30) Priorität: 08.05.2014 DE 102014106489
(43) Veröffentlichungstag der Anmeldung: 11.11.2015
(73) Patentinhaber: B. Braun Avitum AG, 34212 Melsungen (DE)
(72) Erfinder: Alic, Miran, 34212 Melsungen (DE); Strohhöfer, Christof, 34123 Kassel (DE); Janik, Waldemar, 37235 Hessisch Lichtenau (DE)
(74) Vertreter: Winter, Brandl, Fürniss, Hübner, Röss, Kaiser, Polte - Partnerschaft mbB

(56) Entgegenhaltungen:
- WO-A1-96/18096
- WO-A1-2014/026828
- US-A1- 2008 285 011

## Beschreibung

Die vorliegende Erfindung betrifft eine extrakorporale Körperflüssigkeits-Reinigungsmaschine mit einer Vorrichtung zur quantitativen Konzentrationsbestimmung ausgewählter oder auswählbarer Stoffe die aus einem Patientenkörper (natürlich oder künstlich) ausgefiltert wurden, vorzugsweise ausgewählte urämischer Toxine in einer extrakorporalen Blutreinigungsflüssigkeit, insbesondere eine Dialysemaschine zum künstlichen Entfernen von urämischen Toxinen aus dem Blutkreislauf eines Patienten sowie ein Maschinensteuerungsverfahren zum Ermitteln eines Qualitätsmaßes für die Dialyse vorzugsweise der Konzentration ausgewählter Schadstoffe und/oder zum Ermitteln einer entfernten Gesamtmenge an urämischen Toxinen während einer Dialyse (Ermitteln einer Clearance bei einer Dialyse) vorzugsweise bezogen auf ausgewählte/auswählbare, aus dem Patientenblut entfernte Schadstoffe, vorzugsweise Kreatinin und/oder Harnsäure. Die vorliegende Erfindung betrifft weiterhin ein entsprechendes Vorrichtungs-Steuerungsverfahren.

### Hintergrund der Erfindung

Urämische Toxine sind alle Stoffe, die renal ausgeschieden werden müssen. Diese urämischen Toxine akkumulieren sich im Körper eines Menschen/Patienten mit fehlender/verminderter Nierenfunktion. Die Nierenersatztherapie beispielsweise in Form einer Hämodialyse- (HD-) Therapie bietet die Möglichkeit, den Patienten zu behandeln, indem urämische Toxine aus dem intrakorporalen Kreislauf des Patienten in einen Kreislauf mit Dialysierflüssigkeit mittels eines Dialysators überführt werden. Dabei wird das Blut des Patienten über einen extrakorporalen Blutkreislauf in den Dialysator auf dessen Blutseite geleitet und anschließend wieder in den intrakorporalen Blutkreislauf des Patienten eingespeist. Auf der anderen Dialysierflüssigkeitsseite wird der Dialysator über einen Dialysierflüssigkeitseingang mit frischer Dialysierflüssigkeit versorgt, die die urämischen Toxine aufnimmt. Die verbrauchte Dialysierflüssigkeit verlässt schließlich über einen Dialysierflüssigkeitsausgang den Dialysator.

Die Dialysierflüssigkeit ist allgemein eine wässrige Bicarbonat-Pufferlösung mit Eigenschaften ähnlich denen des Blutes in puncto pH-Wert und Elektrolytzusammensetzung. Während einer Hämodialyse-Therapie kommt die Dialysierflüssigkeit mit dem Blut des Patienten über eine Membran innerhalb des Dialysators in Kontakt. Urämische Toxine im Bereich von 18 - 65k Dalton können im Dialysator somit aus dem Blut des Patienten in die Dialysierflüssigkeit diffundieren, womit aus frischer Dialysierflüssigkeit am Eingang die verbrauchte Dialysierflüssigkeit am Ausgang wird.

Die Qualität einer Blutreinigungstherapie, wie Dialyse-Therapie kann abgeschätzt werden, indem die Menge an urämischen Toxinen vor und nach der Behandlung in der Dialysierflüssigkeit erfasst wird. Jedoch ist es nicht nur wichtig, Kenntnisse über die Belastung der verbrauchten Dialysierflüssigkeit an urämischen Toxinen als solches zu erhalten sondern es kann auch die Kenntnis der Zusammensetzung der urämischen Toxine von Interesse sein. Anhand einiger urämischer Toxine wie Kreatinin können nämlich Aussagen beispielsweise über den Ernährungszustand des Patienten gemacht werden. Daher sind sowohl die absolute Menge an entzogenen urämischen Toxinen als auch deren Zusammensetzung von Bedeutung.

Einige urämische Toxine absorbieren Licht im Bereich zwischen 190nm und 350nm. Die Absorption kann genutzt werden, um die Konzentration urämischer Toxine in der ausgangsseitigen Dialysierflüssigkeit zu bestimmen. Die Absorption von Licht kann mit optischen Sensoren gemessen werden, z.B. mit optischen Absorptionssensoren, wie sie aus dem Stand der Technik bereits hinlänglich bekannt sind. Optische Absorptionssensoren sind Vorrichtungen, welche auf Basis einer Transmissionsmessung die Absorption eines Stoffes oder eines Stoffgemisches bestimmen. Unter Zuhilfenahme des Bouguer-Lambert-Beerschen Gesetzes bei einer oder mehreren optischen Wellenlängen kann dabei auch die Konzentration absorbierender Verbindungen (z.B. Kreatinin, Harnsäure usw.) in z.B. optisch klaren Flüssigkeiten bestimmt werden. Obwohl der Absorptionssensor physikalisch eine Transmission misst, wird das Signal oft als dekadisches Absorptionsmaß ausgegeben. Das dekadische Absorptionsmaß ist proportional zum molaren Extinktionskoeffizienten, der Konzentration sowie der optischen Pfadlänge. Allgemein ist die Pfadlänge durch die Sensorgeometrie vorgegeben und somit eine feste Größe. Ähnliches gilt für den molaren Extinktionskoeffizienten, der eine spezifische Stoffeigenschaft ist. Für eine gegebene Wellenlänge ist dieser konstant. Letztendlich ist in einem Sensor das dekadische Absorptionsmaß direkt proportional zu der einzig verbleibenden variablen Größe, nämlich der Konzentration eines Markers. Jedoch gilt das nur für Lösungen mit nur einem gelösten Stoff.

Die Schwierigkeit bei der Untersuchung von Lösungen mit mehreren Komponenten ist jedoch die eindeutige Zuordnung eines Absorptionssignals zur Konzentration einer Komponente. Dies gilt besonders dann, wenn sich die Absorptionsbanden der interessanten Stoffe untereinander (wie beispielsweise Kreatinin und Harnsäure) oder mit uninteressanten Stoffen überlagern.

Wenn es um die therapiebegleitende Bestimmung eines Maßes für die Dialysequalität geht, macht man sich zur Zeit das qualitative Maß Kt/V zunutze mit
- **K** Clearance, (wird z.B. über den Harnstoffgehalt des Blutes vor und nach der Dialyse ermittelt - insbesondere ergibt sich die Clearance aus einem Vergleich der Harnstoffkonzentration am Bluteingang mit der Harnstoffkonzentration am Blutausgang des Dialysators)
- **t** Effektive Dialysezeit in Minuten und
- **V** Der Teil des Körpervolumens, in dem Harnstoff verteilt ist

Da dieses Maß qualitativ ist, erlaubt es keine Aussagen über die tatsächlich entfernten Mengen eines bestimmten Stoffes wie z.B. Kreatinin. Der Markt verlangt aber auch ein quantitatives Maß eines ausgewählten Stoffs, welches einem ermöglicht, weitere Aussagen über den Patienten zu treffen. So kann man weiterhin die Dauer der Dialyse problemlos über die entfernte Gesamtmenge eines Markers steuern, jedoch zusätzlich einen Trend über mehrere Therapien anfertigen, welches erlaubt, z.B. im Fall von Kreatinin den Ernährungszustand des jeweiligen Patienten einzuschätzen.

Grundsätzlich existieren labortechnische Verfahren, insbesondere enzymatische Tests, welche Kratinin-Konzentrationen in Serum bestimmen können. Jedoch reicht eine z.B. ein- oder zweimalige Messung der Kreatininkonzentration im Blut nicht aus, um die entfernte Kreatininmenge quantitativ zu bestimmen. Das liegt im Wesentlichen daran, dass die Entfernungsfunktion, d.h. die Funktion, welche die Konzentration im Blut oder in der Dialysierflüssigkeit während einer Dialyse angibt, unbekannt ist.

Daher ist eine höher frequentierte Messung der Kreatininkonzentration im Blut oder in der verbrauchten Dialysierflüssigkeit erforderlich. Dabei erweist sich die Messung in verbrauchter Dialysierflüssigkeit als vorteilhaft gegenüber der Messung im Blut. Die verbrauchte Dialysierflüssigkeit enthält immer die tatsächlich entfernte Menge an Kreatinin im Gegensatz zu Blut, in dem verschiedene Prozesse für das Nachdiffundieren von Kreatinin sorgen und damit die Bestimmung der tatsächlich entfernten Menge verfälschen.

### Stand der Technik

Aus "Real-time Kt/V determination by ultraviolet absorbance in spent dialysate: technique validation", Kidney International advance online publication, 14- July 2010; doi:10.1038/ki.2010.216, sowie aus "Dialysis dose (Kt/V) and clearance variation sensitivity using measurement of ultraviolet-absorbance (on-line), blood urea, dialysate urea and ionic dialysance", Nephrol Dial Transplant (2006) 21: 2225-2231 doi:10.1093/ndt/gf1147, Advance Access publication 12 April 2006, sind auf UV-Messungen beruhende Konzepte bekannt, mit denen das dimensionslose Verhältnis Kt/V für Harnstoff gemessen wird. Dabei ist K die Clearance in ml/min, V das zu reinigende Volumen (Harnstoffverteilungsvolumen) in ml und t die Dialysedauer in min. Bei den dort beschriebenen Konzepten wird ein Absorptionssignal im Wellenlängenbereich 280nm - 350nm mit der Entnahmerate von Harnstoff korreliert. Durch die Verknüpfung eines Absorptionssignals mit der Harnstoffkonzentration ist diese Vorrichtung in der Lage, durch die Messung eines Absorptionswertes bei einer bestimmten Wellenlänge entsprechende Aussagen über den Wert von Kt/V zu machen.

Ein Nachteil dieses Konzepts besteht darin, dass eine quantitative Angabe der Gesamtmenge des entfernten Harnstoffs nicht möglich ist. Zudem lässt sich keine Aussage über die Zusammensetzung der untersuchten urämischen Toxine machen. Durch die relative Angabe eines Wertes für Kt/V ist die Qualität einer Dialyse nur unzureichend beschrieben.

Aus "Optical measurement of creatinine in spent dialysate", Ruth Tomson, Ivo Fridolin, Frederik Uhlin, Jana Holmar, Kai Lauri and Merike Luman, www.dustri.com/nc/article-response-page.html?artld=10137&doi=, ist eine Methode zur Bestimmung der Kreatinin-Konzentrationen in verbrauchter Dialysierflüssigkeit bekannt. Die Methode basiert auf der Annahme, dass Extinktionswerte verbrauchter Dialysierflüssigkeit bei verschiedenen Wellenlängen genutzt werden können, um ein Vorhersagemodel für die Bestimmung der Kreatinin-Konzentration zu gewinnen.

Mit dem Verfahren nach diesem Stand der Technik kann die erforderliche Genauigkeit (beispielsweise von ca. +/-10%) jedoch nicht sichergestellt werden.

Aus US 7 002 670 B2 ist ein optischer Sensor zur Messung einer bestimmten chemischen Komponente in einer Lösung mit mehreren absorbierenden chemischen Bestandteilen bekannt. Speziell wird ein Verfahren zur Messung von Kreatinin in verbrauchter Dialysierflüssigkeit vor, während und nach einer Dialysebehandlung sowie eine Vorrichtung (optischer Sensor) dafür beschrieben. Die quantitative Bestimmung von Kreatinin erfolgt in einem mehrstufigen Verfahren. In einem ersten Schritt wird die Gesamtabsorption der verbrauchten Dialysierflüssigkeit einschließlich der Absorption von Kreatinin erfasst. Danach wird die Absorption von Kreatinin aus dem Ensemble absorbierender Stoffe selektiv entfernt, indem in einem Reaktor biokatalytische Prozesse (Enzyme) eingesetzt werden. Anschließend wird ein zweites Absorptionssignal erfasst, wobei die Differenz zwischen dem ersten und dem zweiten Absorptionssignal proportional zu der Konzentration von Kreatinin ist.

Ein Nachteil bei diesem Stand der Technik besteht darin, dass Enzyme eingesetzt werden. Enzyme haben den Nachteil, dass u. a. ihre Dosierung und ihre Lagerung/Haltbarkeit überwacht werden müssen.

Aus DE 26 58 101 A1 ist ein Verfahren zur Konzentration von niederdimensionalen Verbindungen in komplexen Medien, vorzugsweise bei medizinischen Behandlungen bekannt. Es wird ein Verfahren beschrieben, welches mit Hilfe der Dialyse einen kleineren Teil eines komplexen Mediums erzeugt. Die Messung des Gehaltes eines Stoffes findet dann in dem kleineren Teil, d.h. in der Dialysierflüssigkeit statt. Insbesondere erfolgt die Messung in der Dialysierflüssigkeit nach oder in Verbindung mit einer chemischen Veränderung der niedrigmolekularen Verbindung mit Hilfe eines oder mehrerer Enzyme.

Ein Problem bei diesem Stand der Technik besteht auch hier darin, dass zur Messung Enzyme mit den vorstehend genannten Nachteilen eingesetzt werden.

Aus US 2008/0015434 A1 ist ein Verfahren bekannt, mit welchem die Auswurfsleistung des Herzens eines Patienten nicht-invasiv während einer Hämodialyse-Therapie bestimmt werden kann. Dazu wird ein fluoreszierender Farbstoff in das kardiovaskuläre System des Patienten gegeben. Der fluoreszierende Farbstoff wird durch eine Strahlungsquelle bei einer ersten Wellenlänge angeregt. Das emittierte Licht wird bei einer zweiten Wellenlänge, die sich von der ersten Wellenlänge unterscheidet, erfasst.

Aus WO 2009/071102 A1 ist eine Vorrichtung bekannt, die eine Küvette zur Aufbewahrung einer biologischen Flüssigkeit umfasst. Die Vorrichtung weist ein optisches Modul zur spektroskopischen Untersuchung der biologischen Flüssigkeit auf, das aus einer Lichtquelle besteht, deren Licht in die Küvette geleitet wird. Das Licht aus der Küvette wird im Anschluss durch einen Detektor erfasst.

Aus WO 2010/024963 A1 ist ein System für die Vorbereitung von Dialysierflüssigkeit bekannt, welches ein Reinigungsmedium für Wasser enthält, eine Vorrichtung für das Pumpen oder Messen von Wasser, eine Heizvorrichtung zum Erhitzen von Wasser, eine Kammer zum Mischen von Wasser und Konzentrat zur Herstellung von frischer Dialysierflüssigkeit, einen Filter für das Filtern von frischer Dialysierflüssigkeit und einen mikroelektromechanischen Sensor (MEMS).

Bei diesem Stand der Technik wird ein Reinigungsmedium oder ein Filter benötigt, so dass der apparative Aufwand relativ groß ist.

Aus DE 10 2011 101 193 A1 ist ein Verfahren zur photometrischen Bestimmung des Harnsäure- und Kreatinin-Gehaltes in einer Flüssigkeit bekannt. Dabei wird in die Flüssigkeit UV-Strahlung bei drei Wellenlängen im Wellenlängenbereich zwischen 235nm und 300nm eingestrahlt und die Strahlenabsorption in der Flüssigkeit bei den vorbestimmten Wellenlängen gemessen. Von den Wellenlängenbereichen befindet sich ein erster in einem Teilbereich zwischen 235nm und 250nm, ein zweiter in einem zweiten Teilbereich zwischen 255 und 270nm und ein dritter in einem dritten Teilbereich zwischen 280nm und 300nm. Es werden der Harnsäure- und Kreatinin-Gehalt sowie optional der Gehalt eines dritten Bestandteils aus den Messergebnissen berechnet.

In EP 1 083 948 B1 wird die Bestimmung des Gehalts einer oder mehrerer kombinierter Substanzen beschrieben. Dazu wird ein spektroskopischer Wert mit dem Fluss der untersuchten Dialysierflüssigkeit multipliziert.

In GB 2 390 420 A wird ein Verfahren zum Bestimmen der Konzentration eines oder mehrerer Stoffe in einer Probe beschrieben. Dazu wird ein für eine Probe aufgestellter Ableitungsalgorithmus eines zweiten Gerätes (z.B. Spektrometer) auf ein erstes Gerät (z.B. Sensor) übertragen. Dadurch wird das erste Gerät in die Lage versetzt, den Gehalt eines Stoffes durch die Messung von Absorbanzwerten bei verschiedenen Wellenlängen zu berechnen.

In US 2008/285 011 A1 wird eine Vorrichtung zur Verwendung bei der Messung des freien Chlorgehalts in einer Lösung von halogeniertem Schwimmbad-/Thermalwasser mit einer pH-Einstellvorrichtung zur Herstellung einer ersten Probe der Lösung mit einem ersten ausgewählten pH-Wert und einer zweiten Probe der Lösung mit einem zweiten ausgewählten pH-Wert beschrieben.

In WO 96/18 096 wird ein Verfahren und eine Vorrichtung zum Bestimmen der Konzentration einer chemischen Zielspezies in einer Probe beschrieben.

In der WO2014 026 828 A1 wird ein Verfahren zur optischen Bestimmung mindestens eines Analyten in einer Probe unter Verwendung mindestens eines Indikatorfarbstoffs, der mindestens eine optische Eigenschaft bei mindestens einer vorgegebenen optischen Wellenlänge in Abhängigkeit von der Konzentration eines gegebenen Analyten ändert.

Bei diesem Stand der Technik werden Standardspektren oder Kurven aus zweiten Geräten zur Berechnung der Konzentration vorausgesetzt.

Damit hat jeder Stand der Technik, der vorstehend erläutert wurde, zumindest einen spezifischen Nachteil. Insbesondere gilt jedoch die Verwendung von Enzymen zur Bestimmung des Kreatinin-Gehalts als nachteilig, da durch sie Lagerungskosten entstehen, ihre Haltbarkeit überwacht werden muss, die Dosierung exakt erfolgen muss und quantifizierbare Messungen überhaupt nur bedingt möglich sind.

Angesichts der vorstehend geschilderten Problematik ist es die grundsätzliche Aufgabe der vorliegenden Erfindung, eine extrakorporale Körperflüssigkeits-Reinigungsmaschine, vorzugsweise Dialysemaschine sowie ein Analyse-/Messverfahren bzw. Maschinensteuerungsverfahren anzugeben, wodurch ein Qualitätsmaß für die Dialyse vorzugsweise die Konzentration ausgewählter Schadstoffe bestimmbar/ermittelbar ist und/oder eine entfernte Gesamtmenge an urämischen Toxinen während einer Dialyse bestimmbar/ermittelbar ist (d. h. Ermitteln einer Clearance bei einer Dialyse durch eine extrakorporale Reinigungsmaschine) und zwar ohne Einsatz von enzymatischen Methoden, Jaffe-Methoden oder sonstigen, nicht während einer üblichen HD-Dialysetherapie vorkommenden Reagenzien. Insbesondere ist es ein Ziel der vorliegenden Erfindung, eine Vorrichtung und ein (Vorrichtungs-Steuerungs-) -verfahren zur quantitativen Konzentrationsbestimmung ausgewählter urämischer Toxine in einer Flüssigkeit, beispielsweise einer vom Körper ausgeschiedenen/entnommenen Flüssigkeit (wie Urin, Blut, Plasma, etc.) oder einer extrakorporalen Blutreinigungsflüssigkeit bereitzustellen auf dessen Basis dann das Qualitätsmaß für die Körperflüssigkeitsreinigung vorzugsweise die Konzentration ausgewählter Schadstoffe bestimmbar/ermittelbar wäre und/oder eine entfernte Gesamtmenge an urämischen Toxinen während einer Körperflüssigkeitsreinigung bestimmbar/ermittelbar wäre (beispielsweise die Clearance bestimmbar wäre).

Diese Aufgabe wird durch eine Vorrichtung mit den Merkmalen des Anspruchs 1 sowie durch ein Verfahren (zum Ermitteln des Qualitätsmaßes gemäß vorstehender Definition) nach Anspruch 12 gelöst. Bevorzugte Ausführungsformen der Erfindung sind Gegenstand der jeweiligen Unteransprüche.

Die Erfindung beruht im wesentlichen darauf, die Konzentration eines ausgewählten und/oder auswählbaren ausgefilterten/dialysierten (Schad-) -stoffs, z.B. eines urämischen Toxins, wie Kreatinin und/oder Harnsäure in einer körperausgeschiedenen/entnommenen Flüssigkeit oder einer verbrauchten Dialysierflüssigkeit mit Hilfe eines optischen Sensors quantitativ zu bestimmen. Hierzu wird beispielsweise während einer extrakorporalen Blutbehandlung/Blutreinigung der pH-Wert der (verbrauchten) Dialysierflüssigkeit (oder der ausgeschiedenen/entnommenen Körperflüssigkeit) verändert und kontinuierlich oder für die unterschiedlichen pH-Werte jeweils ein Extinktionssignal erfasst. Dieses Signal wird dann als Indikator des in der Flüssigkeit /Dialysierflüssigkeit gelösten ausgewählten Stoffs verwendet. Durch Verarbeitung/Vergleich der ermittelten Extinktionssignale in der Flüssigkeit/Dialysierflüssigkeit beispielsweise bei einem ersten pH-Wert und bei einem zweiten pH-Wert wird ein Verarbeitungs-/Vergleichssignal erzeugt, welches von der Konzentration des zu untersuchenden, ausgewählten Stoffs abhängt. Insbesondere ist das Verarbeitungs-/Vergleichssignal ein Differenzsignal, das durch Subtraktion der bei unterschiedlichen pH-Werten aufgenommenen Spektren gebildet wird.

Im Einzelnen wird eine Vorrichtung zur quantitativen Konzentrationsbestimmung ausgewählter oder auswählbarer Stoffe, wie urämische Toxine in einer Flüssigkeit, vorzugsweise einer vom Körper ausgeschiedenen/entnommenen Flüssigkeit oder einer extrakorporalen Blutreinigungsflüssigkeit bereit gestellt, die aufweist:
- einen optischen Absorptionssensor, der dafür angepasst ist, wenigstens ein erstes Extinktionssignal der körperausgeschiedenen/entnommenen Flüssigkeit oder verbrauchten Dialysierflüssigkeit bei wenigstens einer vorgegebenen oder einstellbaren Analysewellenlänge bei einem definierten ersten pH-Wert der körperausgeschiedenen/entnommenen Flüssigkeit oder verbrauchten Dialysierflüssigkeit zu erfassen,
- eine pH-Einstell-/Änderungseinrichtung, die dafür angepasst ist, einen von dem ersten pH-Wert abweichenden zweiten pH-Wert der körperausgeschiedenen/entnommenen Flüssigkeit oder verbrauchten Dialysierflüssigkeit einzustellen,
- den optischen Absorptionssensor, der dafür angepasst ist, wenigstens ein zweites Extinktionssignal der körperausgeschiedenen/entnommenen Flüssigkeit oder verbrauchten Dialysierflüssigkeit mit der wenigstens einen gleichen Analysewellenlänge bei dem zweiten pH-Wert zu erfassen,
- eine Signal-Verarbeitungseinrichtung, die dafür angepasst ist, die beiden Extinktionssignale bei dem ersten und dem zweiten pH-Wert zu verarbeiten, vorzugsweise zu vergleichen und
- eine Ermittlungseinrichtung, die dafür angepasst ist, eine absolute Konzentration mindestens eines ausgewählten oder auswählbaren Stoffs, wie urämische Toxine, in der körperausgeschiedenen/entnommenen Flüssigkeit oder verbrauchten Dialysierflüssigkeit auf der Basis des Ergebnisses der Signal-Verarbeitungseinrichtung zu bestimmen.

Damit macht sich die Erfindung die Abhängigkeit der Absorptionseigenschaften eines ausgewählten und/oder auswählbaren Stoffs vom pH-Wert der Flüssigkeit zunutze, in welcher der Stoff gelöst ist, um zweifelsfrei oder mit größerer Genauigkeit dessen Konzentration (rechnerisch oder tabellarisch) zu bestimmen.

Vorzugsweise hat die Vorrichtung eine Analysewellenlängen-Einstelleinrichtung, die dafür angepasst ist, die Analysewellenlänge auf einen Wert einzustellen, bei welchem der ausgewählte und/oder auswählbare Stoff bei einer Änderung des pH Werts seine Absorptionseigenschaft ändert.

Weiter vorzugsweise ist die Analysewellenlängen-Einstelleinrichtung dafür angepasst, die Analysewellenlänge auf einen Wert einzustellen, bei welchem ein nicht ausgewählter Stoff bei einer Änderung des pH Werts seine Absorbtionseigenschaft nicht oder hinsichtlich der Signal-Verarbeitungseinrichtung vernachlässigbar ändert. Damit muss nicht die Analysewellenlänge verändert werden, wofür ein vergleichsweise teurer Mehr-Wellen-Absorptionssensor erforderlich wäre, sondern es kann mit einem vergleichsweise preisgünstigen Ein-Wellen-Absorptionssensor gearbeitet werden, insbesondere einem Ein-Wellenlängen-Sensor, wobei die Wellenlänge durch die verwendete Lichtquelle entsprechend vorgegeben ist.

Vorzugsweise ist die Vorrichtung dafür angepasst, eine Dialysierflüssigkeit, Blut, Plasma, Urin oder dergleichen medizinische/biologische oder sonstige Lösungsmittel mittels des optischen Absorptionssensors zu analysieren.

Weiter vorzugsweise ist die pH-Einstell-/Änderungseinrichtung dafür angepasst, den ersten pH-Wert auf nährungsweise 7.3 einzustellen. Dieser Wert entspricht in etwa dem von Blut und findet sich im allgemeinen bei herkömmlichen Dialysierflüssigkeiten wieder.

Vorteilhaft wäre es ferner, wenn die pH-Einstell-/Änderungseinrichtung den pH-Wert der körperausgeschiedenen/entnommenen Flüssigkeit oder der Dialysierflüssigkeit durch Anwendung eines elektrolytischen Verfahrens mittels einer Elektrolysezelle (13) oder durch Zugabe von Säuren, Basen und Gasen, wie CO₂ verändert. Dies ermöglicht es nach einer weiteren vorteilhaften Ausgestaltung der Erfindung, dass die pH-Einstell-/Änderungseinrichtung, zumindest den ersten und/oder zweiten pH-Wert über das jeweilige Mischungsverhältnis zwischen der körperausgeschiedenen/entnommenen Flüssigkeit oder der Dialysierflüssigkeit und einem den pH-Wert verändernden Zugabestoff bestimmen kann.

Ferner ist es vorteilhaft, wenn im Fall von sich überlagernden Absorbtionsbanden des ausgewählten Stoffs und zumindest eines weiteren, nicht ausgewählten Stoffs der Absorptionssensor bei einer isosbestischen Analysewellenlänge des nicht ausgewählten Stoffs arbeitet könnte. Auf diese Weise lassen sich Messverfälschungen vermeiden oder vermindern.

Beispielsweise beträgt die Analysewellenlänge näherungsweise 254nm zur Analyse von Kreatinin, entsprechend dem isosbestischen Punkt von Harnsäure und/oder näherungsweise 300nm zur Analyse von Harnsäure. In der Realität könnte zur Analyse von Kreatin beispielsweise eine an sich bekannte Lichtquelle mit einer Lichtwellenlänge von bekanntermaßen 253.7nm verwendet werden, wobei der verwendete Sensor Licht mit einer Wellenlänge von näherungsweise 254nm (konkret zwischen ca. 253nm und ca. 255nm) misst. Derartige Abweichungen von der beschriebenen technischen Lehre entsprechend der real verwendbaren Lichtquellen und Sensoren machen ggf. immer noch vom vorliegenden Erfindungsprinzip Gebrauch und sollen daher von der vorliegenden Offenbarung mit umfasst sein.

Das erfindungsgemäße Maschinensteuerungsverfahren zur quantitativen Konzentrationsbestimmung ausgewählter oder auswählbarer Stoffe, wie urämische Toxine in einer Flüssigkeit, vorzugsweise gelöst in einer vom Körper ausgeschiedenen/entnommenen Flüssigkeit oder einer extrakorporalen Blutreinigungsflüssigkeit sowie vorzugsweise zum Ermitteln eines Qualitätsmaßes für die Dialyse vorzugsweise der Konzentration ausgewählter Schadstoffe und/oder zum Ermitteln einer entfernten Gesamtmenge an urämischen Toxinen während einer Dialyse mit einer Dialysemaschine weist die folgenden Schritte auf:
Erfassen wenigstens eines ersten Extinktionssignals einer vom Körper ausgeschiedenen/entnommenen Flüssigkeit oder einer verbrauchten Dialysierflüssigkeit bei wenigstens einer vorgegebenen oder ausgewählten Analysewellenlänge bei einem ersten vorzugsweise vorab eingestellten pH-Wert der körperausgeschiedenen/entnommenen Flüssigkeit oder der verbrauchten Dialysierflüssigkeit durch einen optischen Absorptionssensor,
Einstellen eines von dem ersten pH-Wert abweichenden zweiten pH-Werts der körperausgeschiedenen/entnommenen Flüssigkeit oder der Dialysierflüssigkeit durch eine pH-Einstelleinrichtung,
Erfassen wenigstens eines zweiten Extinktionssignals der körperausgeschiedenen/entnommenen Flüssigkeit oder der verbrauchten Dialysierflüssigkeit bei der wenigstens einen vorgegebenen oder ausgewählten Analysewellenlänge bei dem zweiten pH-Wert durch den Absorptionssensor,
Verarbeiten der beiden Extinktionssignale bei dem ersten und dem zweiten pH-Wert vorzusweise durch Differenzbildung und Ermitteln vorzugsweise durch Berechnen oder tabelarisches Bestimmen einer absoluten Konzentration mindestens eines in der körperausgeschiedenen/entnommenen Flüssigkeit oder der verbrauchten Dialysierflüssigkeit gelösten ausgewählten Stoffs auf der Basis des Verarbeitungsergebnisses.

Vorzugsweise wird im Fall sich überlagernder Absorbtionsbanden des ausgewählten Stoffs und wenigstens eines weiteren, nicht ausgewählten Stoffs die wenigstens eine vorgegebene Analysewellenlänge als eine isosbestische Analysewellenlänge bezüglich des nicht ausgewählten Stoffs bestimmt.

Weiter vorzugsweise wird die wenigstens eine vorgegebene Analysewellenlänge auf näherungsweise 254nm zur Analyse von Kreatinin eingestellt, entsprechend dem isosbestischen Punkt von Harnsäure und/oder auf näherungsweise 300nm eingestellt zur Analyse von Harnsäure.

Das erfindungsgemäße Verfahren zum Ermitteln des ausgewählten Stoffs und ggf. zum Ermitteln des Qualitätsmaßes für die Dialyse vorzugsweise der Konzentration ausgewählter Schadstoffe und/oder zum Ermitteln einer entfernten Gesamtmenge an urämischen Toxinen während einer Dialyse (Clearance der Niere oder bei der Dialyse der Dialysemaschine), weist in anderen Worten ausgedrückt die Schritte auf:
- Erfassen wenigstens eines ersten Extinktionssignals der körperausgeschiedenen/entnommenen Flüssigkeit oder einer verbrauchten Dialysierflüssigkeit bei wenigstens einer vorgegebenen Analysewellenlänge bei einem definierten ggf. vorab eingestellten ersten pH-Wert der Flüssigkeit/Dialysierflüssigkeit durch einen (optischen) Absorptionssensor,
- Ändern des ersten pH-Werts auf einen hiervon abweichenden zweiten pH-Wert der körperausgeschiedenen/entnommenen Flüssigkeit oder der Dialysierflüssigkeit durch eine pH-Einstell-/Änderungseinrichtung,
- Erfassen wenigstens eines zweiten Extinktionssignals der körperausgeschiedenen/entnommenen Flüssigkeit oder verbrauchten Dialysierflüssigkeit bei der wenigstens einen vorgegebenen Analysewellenlänge bei dem zweiten pH-Wert durch den Absorptionssensor,
- Verarbeiten/Vergleichen der wenigstens zwei Extinktionssignale zumindest bei dem ersten und dem zweiten pH-Wert und Ermitteln einer absoluten Konzentration mindestens eines in der körperausgeschiedenen/entnommenen Flüssigkeit (Urin, Blut, Plasma, etc.) oder in der Dialysierflüssigkeit gelösten ausgewählten Stoffs.

Damit lässt sich das erfindungsgemäße Steuerungsverfahren wie folgt konkreter umschreiben, wobei an dieser Stelle darauf hinzuweisen ist, dass sowohl die Vorrichtung als auch das Verfahren nicht nur bei einer Patientendialyse sondern auch bei anderen medizinischen oder nicht medizinischen Vorgängen zur Ermittlung der Konzentration von in Flüssigkeit gelöster Stoffe prinzipiell anwendbar ist:

In einem ersten Schritt wird zunächst ausgewählt, welcher (Schad-) -stoff (dessen Konzentration), z. B. die Kreatininkonzentration untersucht werden soll.

In einem zweiten Schritt wird ein Wellenlängenbereich definiert, in welchem der zu untersuchende (Schad-) -stoff eine Absorptions-/Absorbanzeigenschaft zeigt, die sensorisch (optisch) detektierbar ist.

In einem dritten Schritt wird überprüft, ob - und wenn ja - mit welchem(n) anderen (Schad-) -stoff(en) ggf. eine Überlagerung der jeweiligen Absorptionsbanden (Wellenlängen) vorliegt, wodurch eine Zuordnung eines sensorisch erhaltenen aktuellen Absorptionssignals zu einer entsprechenden Konzentration des zu untersuchenden (Schad-) -stoffs erschwert/unmöglich gemacht wird.

In einem vierten Schritt wird die zu verwendende Wellenlänge in einem isobestischen Punkt des/der anderen, nicht ausgewählten (Schad-) -stoffe(s) bestimmt, sodass im Fall einer pH-Änderung der körperausgeschiedenen/entnommenen Flüssigkeit oder Dialysierflüssigkeit nur der zu untersuchende, ausgewählte (Schad-) - stoff seine Absorptionseigenschaft ändert (und die anderen, nicht ausgewählten (Schad-) -stoffe nicht/geringfügig).

In einem fünften Schritt erfolgt eine Messung der körperausgeschiedenen/entnommenen Flüssigkeit/verbrauchten Dialysierflüssigkeit mittels eines (optischen) Sensors (UV-Sensors), der auf die in Schritt vier bestimmte Wellenlänge eingestellt ist, zumindest bei einem 1. (ausgewählten oder vorbestimmten) pH-Wert der körperausgeschiedenen/entnommenen Flüssigkeit oder Dialysierflüsigkeit, um einen ersten Messwert zu erhalten.

In einem sechsten Schritt erfolgt eine Messung der körperausgeschiedenen/entnommenen Flüssigkeit/verbrauchten Dialysierflüssigkeit mittels des (optischen) Sensors (UV-Sensors), der auf die in Schritt vier bestimmte Wellenlänge eingestellt ist, zumindest bei einem 2. (ausgewählten oder vorbestimmten) pH-Wert der körperausgeschiedenen/entnommenen Flüssigkeit/Dialysierflüsigkeit, um zumindest einen zweiten Messwert zu erhalten (oder kontinuierliche Messung).

In einem siebten Schritt wird ein Verarbeitungs-, vorzugsweise Differenz-Signal (proportional zur Konzentration des zu untersuchenden (Schad-) -stoffs) zwischen den unterschiedlichen Messwerten gebildet.

In einem achten Schritt wird schließlich das Verarbeitungs-Signal einer entsprechenden Konzentration des ausgewähltens Stoffs zugeordnet.

Bevorzugte Ausführungsformen des erfindungsgemäßen Verfahren weisen als eines oder als Kombination mehrerer Merkmale auf, dass:
- die wenigstens eine vorgegebene Analyse-/Meßwellenlänge eine isosbestische Analysewellenlänge ist, bei der das Extinktionssignal eines nicht gesuchten Stoffes mit sich überlagernden pH-spezifischen Absorptionseigenschaften unabhängig vom pH-Wert ist;
- die wenigstens eine vorgegebene Analysewellenlänge 254nm beträgt, entsprechend dem isosbestischen Punkt der Harnsäure;
- der pH-Wert der ausgangsseitigen Dialysierflüssigkeit durch Elektrolyse, durch Zugabe einer Säure, einer Base oder eines Gases, insbesondere CO2, geändert wird;
- mehrere unterschiedliche Analysewellenlängen verwendet werden zur Bestimmung verschiedener Stoffe.

An dieser Stelle seien folgende Konstellationen beispielhaft genannt:
Wenn bevorzugt die Konzentration von Kreatinin analysiert/gemessen werden soll, sollte die Mess-Wellenlänge des optischen Sensors beispielsweise auf 254nm eingestellt sein, da dort der Harnsäureanteil (im wesentlichen) unschädlich für das Messergebnis ist, weil diese Wellenlänge den isosbestischen Punkt der Harnsäure darstellt. Wird bei dieser Wellenlänge der pH-Wert der körperausgeschiedenen/entnommener Flüssigkeit/Dialysierflüssigkeit geändert, ändert sich die Absorptionseigenschaft (Absorbanz) von Kreatinin, nicht aber von Harnsäure. Damit kann die Kreatinin-Konzentration detektiert und bestimmt werden. Wenn bevorzugt die Konzentration von Harnsäure analysiert/gemessen werden soll, sollte die Mess-Wellenlänge des optischen Sensors beispielsweise auf 300nm eingestellt sein, da dort der Kreatininanteil unschädlich für das Messergebnis ist.

Die entsprechende erfindungsgemäße Vorrichtung zur quantitativen Konzentrationsbestimmung ausgewählter urämischer Toxine in einer Flüssigkeit, beispielsweise einer vom Körper ausgeschiedenen Flüssigkeit (wie Urin) oder einer extrakorporalen Blutreinigungsflüssigkeit beispielsweise eine Dialysemaschine umfasst:
- einen Absorptionssensor zum Erfassen wenigstens eines ersten Extinktionssignals der körperausgeschiedenen Flüssigkeit oder der verbrauchten Dialysierflüssigkeit bei wenigstens einer vorgegebenen Analysewellenlänge bei einem von der körperausgeschiedenen Flüssigkeit oder Dialysierflüssigkeit definierten ggf. einstellbaren ersten pH-Wert,
- eine pH-Einstelleinrichtung zum Einstellen eines von dem ersten pH-Wert der körperausgeschiedenen Flüssigkeit oder der verbrauchten Dialysierflüssigkeit abweichenden zweiten pH-Wertes,
- den Absorptionssensor zum Erfassen wenigstens eines zweiten Extinktionssignals der körperausgeschiedenen Flüssigkeit oder der verbrauchten Dialysierflüssigkeit bei der wenigstens einen vorgegebenen Analysewellenlänge in Abhängigkeit von dem zweiten pH-Wert,
- eine Vergleichseinrichtung zum Vergleichen der beiden Extinktionssignale bei dem ersten und dem zweiten pH-Wert und
- eine Ermittlungseinrichtung zum Ermitteln einer absoluten Konzentration mindestens einer in der körperausgeschiedenen Flüssigkeit /Dialysierflüssigkeit gelösten Substanz auf der Grundlage der Ergebnisses der Vergleichseinrichtung.

Ein Vorteil der Erfindung besteht darin, dass eine optische Auswertung einfach in ein Flusssystem integrierbar ist und eine hohe Messgenauigkeit bietet. So kommt die Erfindung bei der Messung beispielsweise von Kreatinin mit Hilfe eines optischen Sensors ohne Enzyme aus, die teurer, schwierig zu dosieren sind und eine kurze Haltbarkeit aufweisen. Die quantitative Messung beispielsweise von Kreatinin oder Harnsäure wird auch im Verlauf einer Dialysetherapie ermöglicht. Auch ermöglich die Erfindung, dass ein günstiger Sensor eingesetzt werden kann, der für eine Wellenlänge ausgelegt ist. Insbesondere lässt sich mit einem optischen Sensor in einem sehr kleinen Wellenlängenbereich aus einem dort vorherrschenden Extinktionssignal eine Kreatininkonzentration berechnen.

Weitere Merkmale und Vorteile der Erfindung ergeben sich aus der folgenden Beschreibung von bevorzugten Ausführungsformen, bei der Bezug genommen wird auf die beigefügte Zeichnung.
Fig. 1 zeigt schematisch eine erste Ausführungsform der erfindungsgemäßen Dialysevorrichtung.
Fig. 2 zeigt schematisch eine zweite Ausführungsform der erfindungsgemäßen Dialysevorrichtung.
Fig. 3 zeigt schematisch eine dritte Ausführungsform der erfindungsgemäßen Dialysevorrichtung.
Fig. 4 zeigt ein Extinktionsspektrum von unverbrauchter Dialysierflüssigkeit im Vergleich zu dem Extinktionsspektrum von Kreatinin.
Fig. 5 zeigt das Extinktionsverhalten von Kreatinin bei einem pH-Wert der Dialysierflüssigkeit von um 7,3 bzw. um 4.
Fig. 6 zeigt Differenzextinktiongsspektren von Harnsäure und Kreatinin als Verdünnungsreihe sowie die Extinktionsspektren bei einem pH-Wert von 7,3.

Die Erfindung beruht darauf, mit Hilfe der Änderung des pH-Wertes beispielsweise von verbrauchter Dialysierflüssigkeit die Absorptionseigenschaften einer Auswahl von Stoffen, u. a. Kreatinin und/oder Harnsäure, zu ändern. Aus der Messung der Absorption zumindest bei zwei verschiedenen pH-Werten wird ein Differenzsignal generiert. Dieses Differenzsignal enthält nur noch Beiträge von weniger Stoffen als das ursprüngliche Absorptionssignal, so dass sich die Bestimmung der Konzentration einzelner Stoffe vereinfacht. Insbesondere werden die Sensorparameter so gewählt, dass die Kreatininkonzentration in der verbrauchten Dialysierflüssigkeit bestimmbar ist.

Kreatinin ist nicht der einzige Stoff, der durch die Änderung des pH-Wertes eine Veränderung der Absorptionseigenschaften erfährt. Zwar ist die Anzahl der urämischen Toxine, welche eine starke Änderung der Absorptionseigenschaften zeigen, deutlich geringer als die Gruppe aller absorbierender Stoffe in verbrauchter Dialysierflüssigkeit, was die Zuordnung stark vereinfacht. Jedoch gibt es urämische Toxine, wie zum Beispiel Harnsäure, welche ihre Absorptionseigenschaften im gleichen pH- und Wellenlängenbereich wie Kreatinin ändern.

Um die Differenzsignale zu vereinfachen, werden Messungen vorzugsweise in isosbestischen Punkten vorgenommen. Isosbestische Punkte sind Wellenlängenpositionen bei denen sich der Wert der Extinktion vor und nach der Änderung des pH-Wertes nicht ändert, obwohl der entsprechende Stoff bei anderen Wellenlängen durchaus eine Abhängigkeit der Absorption vom pH-Wert aufweist. Da ein isosbestischer Punkt von Harnsäure in dem Wellenlängenbereich liegt, in dem das Differenzsignal von Kreatinin ausgeprägt ist, kann dort das Differenzsignal gänzlich Kreatinin zugeschrieben werden.

Eine erste Ausführungsform der erfindungsgemäßen Dialysevorrichtung welche beispielhaft beschrieben wird sieht vor, dass eine erste Pumpe frische Dialysierflüssigkeit zum Eingang eines Dialysators fördert. Im Dialysator kommt es zum Transfer von urämischen Toxinen aus dem Blut des Patienten in die Dialysierflüssigkeit. Infolgedessen wird aus frischer Dialysierflüssigkeit verbrauchte Dialysierflüssigkeit. Die verbrauchte Dialysierflüssigkeit verlässt den Dialysator an dessen Ausgang und wird innerhalb eines dialysierflüssigkeitsseitigen Schlauchsystems in Richtung Verwurf transportiert. Die verbrauchte Dialysierflüssigkeit kann dabei verschiedene Vorrichtungen passieren, wie zum Beispiel eine Bilanzkammer oder eine optionale Mischkammer. Entscheidend dabei ist, dass es zu einem Zeitpunkt vor der Änderung des pH-Wertes an einem optischen Sensor vorbeifließt, welcher zumindest ein erstes Extinktionssignal von verbrauchter Dialysierflüssigkeit erfassen kann/erfasst. In einem zweiten Schritt wird vor dem optischen Sensor durch eine zweite Pumpe eine pH-Wert ändernde Substanz in die verbrauchte Dialysierflüssigkeit eingeleitet. Der angeschlossene optische Sensor (Absorptionssensor) liefert dann zumindest ein zweites Extinktionssignal bei einem zweiten pH-Wert, der sich vom ersten pH-Wert unterscheidet. Aus den zumindest zwei erhaltenen Extinktionswerten kann dann die Konzentration eines Stoffes beispielsweise rechnerisch oder tabellarisch ermittelt werden.

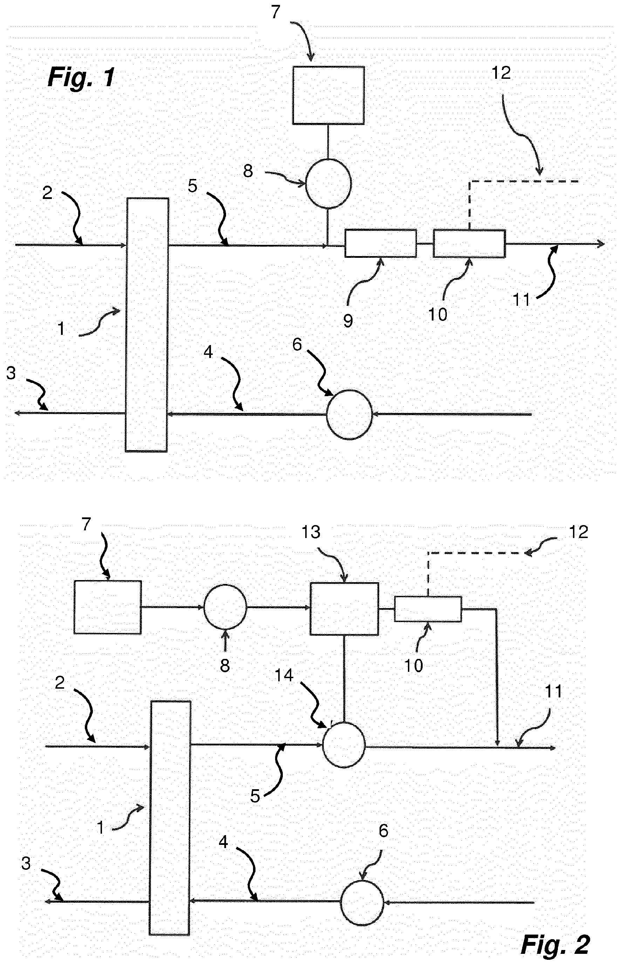

Im Einzelnen ist dies in Fig. 1 dargestellt. Fig. 1 zeigt schematisch in einer ersten Ausführungsform der Erfindung einen Dialysator 1 mit einem Bluteingang 2 und einem Blutausgang 3. Über den Bluteingang 2 wird Blut aus dem Kreislauf des (nicht gezeigten) Patienten in den Dialysator 1 geleitet, wo es durch Diffusion und/oder Konvektion bedingt bestimmte Stoffe (urämische Toxine) im Blut des Patienten durch eine (nicht gezeigte) Membran hindurch an eine Dialysierflüssigkeit abgibt. Die Dialysierflüssigkeit wird über einen Dialysierflüssigkeitseingang 4 in den Dialysator gepumpt und über einen Dialysierflüssigkeitsausgang 5 wieder entfernt. Hierfür ist eine Dialysierflüssigkeitspumpe 6 vorgesehen.

Bei einer Dialysebehandlung ist es vorteilhaft und ggf. notwendig, wenn die Konzentration der aus dem Blut in die Dialysierflüssigkeit übergetretenen Stoffe überwacht wird. Die Erfindung nutzt dazu die mit einer pH-Wert-Änderung der ausgangsseitigen, d.h. mit urämischen Toxinen befrachteten Dialysierflüssigkeit einhergehende Veränderung optischer Parameter beispielsweise infolge Änderungen von Absorptionseigenschaften bestimmter darin gelöster Stoffe. Um den pH-Wert der Dialysierflüssigkeit manipulieren zu können, ist vorzugsweise ein Substanzbehälter 7 mit einer pH-Wert-verändernden Substanz vorgesehen. Über eine Substanzpumpe 8 wird die Substanz der ausgangsseitigen Dialysierflüssigkeit beigemengt. Dies erfolgt beispielhaft entweder direkt oder in einer optionalen Mischstrecke 9 der erfindungsgemäßen Dialysatorvorrichtung, wobei auch andere Maßnahmen getroffen werden können.

Die Veränderung der optischen Eigenschaften der so manipulierten verbrauchten Dialysierflüssigkeit werden in einem optischen Sensor 10 erfasst. Der optische Sensor 10 ist insbesondere ein Absorptions- oder Extinktionssensor. Anschließend wird die verbrauchte Dialysierflüssigkeit in einem sog. Verwurf 11 entsorgt. Die Daten zumindest aus dem optischen Sensor 10 werden über eine Kommunikationsschnittstelle 12 an eine (nicht gezeigte) Recheneinheit zur weiteren Verarbeitung ausgegeben.

Um beispielsweise ein Qualitätsmaß für die Dialyse vorzugsweise der Konzentration ausgewählter Schadstoffe und/oder eine entfernte Gesamtmenge an urämischen Toxinen während einer Dialyse (Clearance bei einer Dialyse) zu ermitteln und damit die Qualität, bzw. den Fortgang der Dialyse beurteilen zu können, wird zumindest ein pH-Wert der ausgangsseitigen Dialysierflüssigkeit durch die pH-Einstelleinrichtung eingestellt, die in der Ausführungsform nach Fig. 1 eine optionale Mischstrecke 9 umfasst. Das Extinktionssignal der ausgangsseitigen Dialysierflüssigkeit wird in dem optischen Sensor 10 erfasst und ausgewertet. Dabei ist die Ausführungsform nicht auf nur eine Absorptionsmessung und einen pH-Wert der Dialysierflüssigkeit beschränkt. Die Absorptionsmessung kann ebenso gut bei mehreren Analysewellenlängen des optischen Sensors 10 erfolgen, und die Absorptionsmessung wird dabei für mehrere pH-Werte der Dialysierflüssigkeit durchgeführt. Die Extinktionssignale bei einer Wellenlänge werden für jeden eingestellten pH-Wert verglichen, und aus dem Vergleich wird eine absolute Konzentration einer in der Dialysierflüssigkeit gelösten Substanz ermittelt. Der Vergleich beinhaltet dabei insbesondere eine Subtraktion der Absorptionswerte voneinander. Alternativ kann jedoch statt einer Differenz ein Quotient der Absorptionswerte gebildet werden.

Die obige Vorgehensweise kann optional auf die folgende Art modifiziert werden. Eine erste Pumpe fördert frische Dialysierflüssigkeit zum Eingang des Dialysators. Im Dialysator kommt es zum Transfer von urämischen Toxinen aus dem Blut des Patienten in die Dialysierflüssigkeit. Infolgedessen wird aus frischer Dialysierflüssigkeit verbrauchte Dialysierflüssigkeit. Die verbrauchte Dialysierflüssigkeit verlässt den Dialysator an dessen Ausgang und wird innerhalb eines Schlauchsystems in Richtung Verwurf transportiert. An einem optionalen Drei-Wege-Ventil wird die Richtung des Flusses bestimmt. Zu einem ersten Zeitpunkt wird das Ventil auf eine solche Art angesteuert, dass verbrauchte Dialysierflüssigkeit in einen Behälter umgeleitet wird. Die aus dem Behälter austretende Flüssigkeit durchfließt einen optischen Sensor, der ein erstes Extinktionssignal aufnimmt. Danach wird das Drei-Wege-Ventil umgeschaltet, was dazu führt, dass der Fluss verbrauchter Dialysierflüssigkeit direkt in den Verwurf geleitet wird. Im nächsten Schritt fördert eine Pumpe eine pH-Wert-ändernde Flüssigkeit in den Behälter. Danach fördert die Pumpe die Flüssigkeit mit veränderten pH-Wert aus dem Behälter zum optischen Sensor, der ein zweites Extinktionssignal aufzeichnet. Aus den beiden erhaltenen Werten wird die Konzentration eines Stoffes ermittelt.

Die Veränderung des pH-Wertes kann auch auf anderen Wegen erfolgen, zum Beispiel durch die Änderung der Zusammensetzung der Dialysierflüssigkeit an sich. In diesem Falle würde der Behälter mit der pH-Wert ändernden Substanz entfallen und stattdessen die pH-Wert-ändernde Substanz als Bolus im Fluss der verbrauchten Dialysierflüssigkeit mitgeführt werden. Dies kann auf verschiedene Wege erreicht werden, wie z.B. im Bypassmodus (nicht abgebildet), bei dem Dialysierflüssigkeitsausgang und Dialysierflüssigkeitseingang direkt miteinander verbunden werden. Der Bolus kann dann durch entsprechende Schaltung des optionalen Drei-Wege Ventils aus dem Fluss der Dialysierflüssigkeit selektiert werden und in den Behälter zum Anpassen des pH-Wertes umgeleitet werden. Die so entstandene Flüssigkeit lässt am optischen Sensor wiederum ein zweites Extinktionssignal entstehen, das zur Berechnung einer Konzentration herangezogen wird.

Die entsprechende Vorrichtung ist in Fig. 2 gezeigt. Über den Bluteingang 2 wird Blut aus dem Kreislauf des (nicht gezeigte) Patienten in den Dialysator 1 geleitet, wo es durch Diffusion und/oder Konvektion bedingt bestimmte Stoffe durch eine (nicht gezeigte) Membran hindurch an eine Dialysierflüssigkeit abgibt. Die Dialysierflüssigkeit wird über einen Dialysierflüssigkeitseingang 4 in den Dialysator gepumpt und über einen Dialysierflüssigkeitsausgang 5 wieder entfernt. Hierfür ist vorzugsweise eine Dialysierflüssigkeitspumpe 6 vorgesehen.

Ein Substanzbehälter 7 versorgt über eine Substanzpumpe 8 einen Anpassungsbehälter 13 zur Anpassung des pH-Wertes seines Inhalts mit einer pH-Wert-verändernden Substanz. Die optischen Eigenschaften des Inhalts des Anpassungsbehälters 13 werden in einem optischen Sensor 10 erfasst und über eine Kommunikationsschnittstelle 12 an eine (nicht gezeigte) Recheneinheit zur weiteren Verarbeitung ausgegeben. Die Dialysierflüssigkeit wird im Verwurf 11 entsorgt.

Die wechselnde Versorgung des Anpassungsbehälters 13 mit verbrauchter Dialysierflüssigkeit, das Ablassen des Anpassungsbehälters und die Entsorgung seines Inhalts in den Verwurf erfolgt mit einem Drei-Wege-Ventil 14. Damit ist die Ausführungsform der erfindungsgemäßen Dialysevorrichtung besonders einfach steuerbar aufgebaut. Mit einem derartigen Drei-Wege-Ventil lässt sich darüber hinaus auch auf einfache Art und Weise der (nicht dargestellte) Bypassmodus verwirklichen, bei dem Dialysierflüssigkeitsausgang und Dialysierflüssigkeitseingang direkt miteinander verbunden werden.

Eine weitere Modifikation des Aufbaus nach Fig. 1 bzw. 2 besteht darin, dass die pH-Wertänderung durch Elektrolyse erfolgt. Der Aufbau der erfindungsgemäßen Dialysevorrichtung bei diesem Ansatz gleicht dem obigen Ausführungsbeispiel nach Fig. 2 bis zum Erreichen der verbrauchten Dialysierflüssigkeit am Drei-Wege Ventil 14. Dort wird die verbrauchte Dialysierflüssigkeit in einen Behälter gebracht, der gleichzeitig eine Elektrolysezelle ist. Der Absorptionssensor erfasst ein erstes Extinktionssignal vor der Änderung des pH-Wertes und ein zweites Extinktionssignal, nachdem in der Elektrolysezelle ein bestimmter pH-Wert eingestellt worden ist. Aus den Extinktionssignalen wird die Konzentration des gewünschten Stoffes ermittelt.

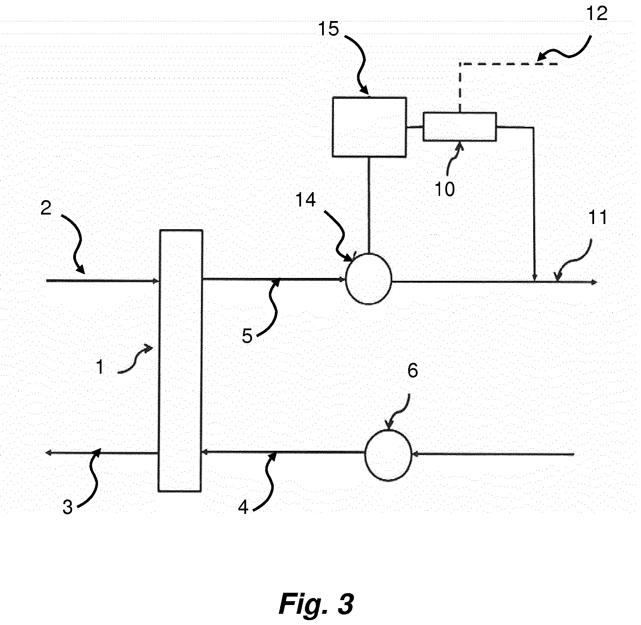

Der Aufbau in Fig. 3, mit dem der pH-Wert durch Elektrolyse verändert wird, weist folglich anstelle von Vorratsbehältern für pH-Wert verändernde Substanzen einen Elektrolysebehälter 15 auf, der zur Anpassung des pH-Wertes mittels Elektrolyse dient. Damit ist die Ausführungsform nach Fig. 3 besonders kompakt aufgebaut.

Die Ermittlung des Qualitätsmaßes für die Dialyse vorzugsweise der Konzentration ausgewählter Schadstoffe und/oder der entfernten Gesamtmenge an urämischen Toxinen während einer Dialyse (Clearance) stützt sich auf einen bestimmten im Blut bzw. in der Dialysierflüssigkeit gelösten Stoff. Dementsprechend wird der optische Sensor auf diesen Stoff eingestellt. Für die Messung von Kreatinin durch eine Vorrichtung nach Fig. 1, 2 oder 3 wird die Sensorwellenlänge für die Messung von Kreatininkonzentrationen auf näherungsweise 254nm gesetzt. Durch die Wahl dieser Wellenlänge kann Harnsäure bei der Berechnung der Kreatininkonzentrationen vernachlässigt werden.

Voraussetzung für die Messung mit dieser Wellenlänge ist eine vorzugsweise symmetrische Lichtquelle mit der Zentralwellenlänge von 254nm +/- 5nm. Das kann durch den Einsatz von LEDs oder Lampen erreicht werden. Insbesondere Hg-Niederdrucklampen zeichnen sich durch eine schmalbandige Linie bei 253.7nm aus und stellen damit in Kombination mit entsprechenden Filtern die optimale Lichtquelle zur Bestimmung der Kreatinin-Extinktion dar. Statt einer symmetrischen Lichtquelle können breitbandige Lichtquellen in Verbindung mit entsprechend schmalbandigen optischen Filtern bzw. Monochromatoren eingesetzt werden.

Die Erfindung ist nicht auf die Messung von Kreatinin beschränkt. So kann mit einer Vorrichtung nach Fig. 1, 2 oder 3 auch Harnsäure erfasst werden. Die pH-Wertabhängige Verschiebung der Extinktion von Harnsäure kann in vielen Wellenlängenbereichen beobachtet werden. Der Bereich zwischen 290nm und 310nm eignet sich für die Unterscheidung zu anderen Substanzen in der verbrauchten Dialysierflüssigkeit besonders gut. Der Bereich zwischen 260nm und 290nm ist ebenfalls möglich. Bei der Untersuchung von Harnsäuregehalt in der Dialysierflüssigkeit kann eine Lichtquelle eingesetzt werden, z.B. eine entsprechende LED, die eine Halbwertsbreite von maximal 10nm aufweist.

Die Extinktionsmessungen bei Kreatinin und Harnsäure werden im folgenden anhand von Extinktionsspektren erläutert, die nach dem erfindungsgemäßen Verfahren gewonnen wurden.

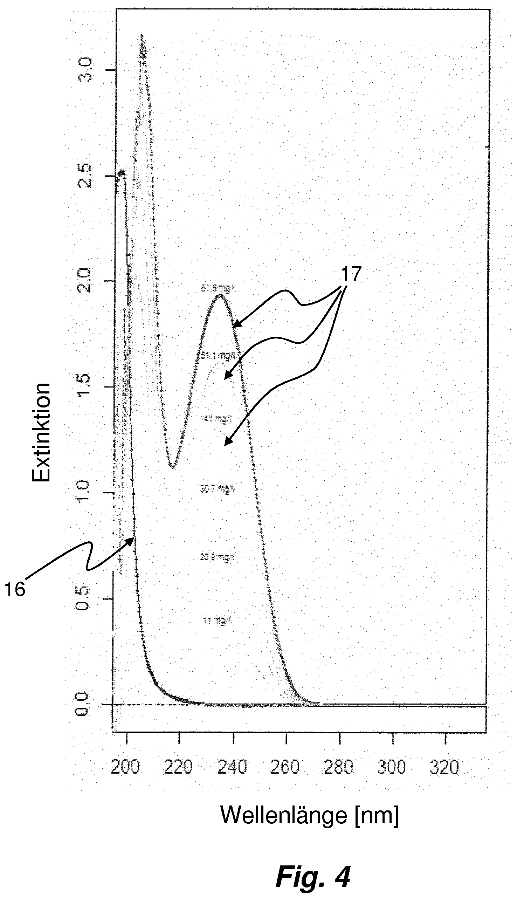

Urämische Toxine absorbieren Licht im Bereich zwischen 190nm - 400nm. Fig. 4 zeigt ein Extinktionsspektrum 17 von ausgangsseitiger Dialysierflüssigkeit für mehrere unterschiedliche Konzentrationswerte von Kreatinin. Zum Vergleich ist in Fig. 4 im gleichen Bereich ein Extinktionsspektrum 16 für die eingangsseitige Dialysierflüssigkeit gezeigt.

In der Praxis setzt sich das Extinktionsspektrum 17 von verbrauchter Dialysierflüssigkeit jedoch aus mehr als nur dem Kreatinin-Spektrum zusammen. Überlagerungen von Extinktionsspektren anderer urämischer Toxine erschweren die eindeutige Zuweisung einer Extinktion zu einzelnen urämischen Toxinen wie zum Beispiel Kreatinin oder Harnsäure.

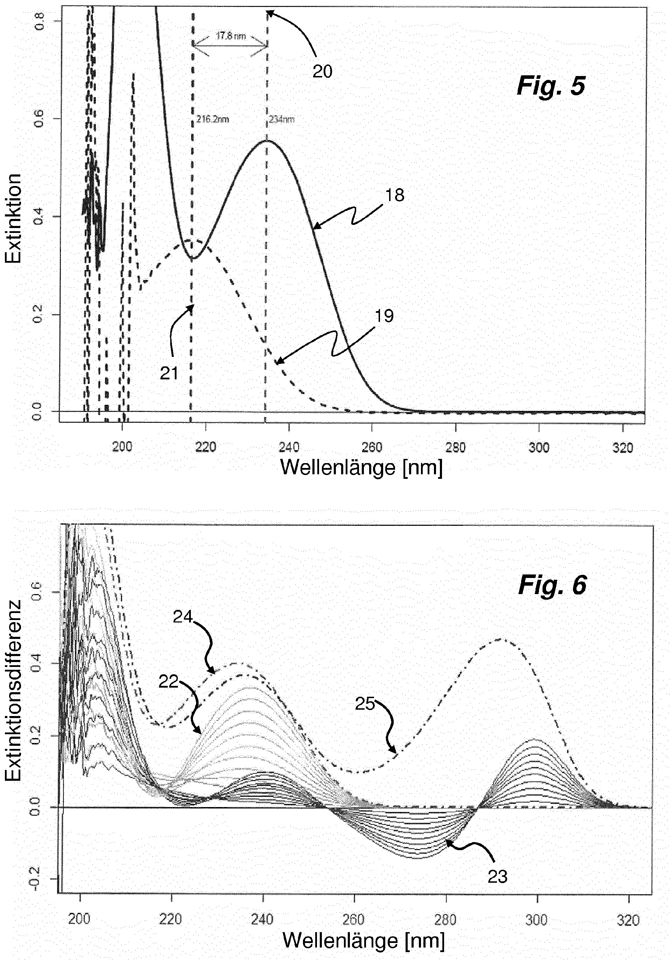

Darüber hinaus wird die Form des Spektrums auch durch den eingestellten pH-Wert verändert. Das bedeutet, dass Kreatinin, gelöst in Dialysierflüssigkeit, ein Extinktionsspektrum 17 aufweist, welches vom pH-Wert der Dialysierflüssigkeit abhängt. Fig. 5 zeigt eine Extinktion 18 von Kreatinin bei einem pH-Wert der Dialysierflüssigkeit von ∼ 7,3 (durchgezogene Linie) und eine Extinktion 19 von Kreatinin bei einem pH-Wert der Dialysierflüssigkeit von ∼4 (gestrichelte Linie). Die Extinktion 18 von Kreatinin bei einem pH-Wert der Dialysierflüssigkeit von -7,3 weist ein langwelliges lokales Maximum 20 bei etwa 234nm auf, die Extinktion 19 von Kreatinin bei einem pH-Wert der Dialysierflüssigkeit von cirka 4 weist ein langwelliges lokales Maximum 21 bei etwa 216nm auf. Die Wellenlängen der Maxima 20, 21 des Extinktionsspektrums sind als gestrichelte vertikale Linien dargestellt. Der Abstand der Maxima 20 und 21 für den pH-Wert von 7,3 bzw. cirka 4 beträgt etwa 18nm. Gleichzeitig nimmt mit dem pH-Wert die Extinktion im jeweiligen Maximum um etwa 40% ab.

Eine Änderung der Extinktion in Abhängigkeit vom pH-Wert tritt nicht nur bei Kreatinin auf. Aus einer großen Menge absorbierender Substanzen, welche in verbrauchter Dialysierflüssigkeit vorkommen können, wurde bisher allerdings nur ein weiteres urämische Toxin identifiziert, dessen Extinktionssignal sich in vergleichbarer Weise in Abhängigkeit von dem pH-Wert ändert wie Kreatinin, nämlich Harnsäure.

Die Konzentrationsmessung dieser beiden Stoffe erfolgt erfindungsgemäß durch die Bestimmung der Differenzspektren an mindestens zwei verschiedenenpH-Werten bzw. Extinktionswerten. Grundsätzlich können für die Berechnung der stoffspezifischen Extinktion alle Wellenlängenpositionen verwendet werden, bei denen die Extinktionsdifferenz größer als Null ist. Tatsächlich hängt die Wahl der Wellenlängenposition von der Verfügbarkeit entsprechender Lichtquellen ab. Vorzugsweise wird für die Bestimmung der Extinktion von Kreatinin ein Sensor verwendet, welcher in dem isosbestischen Punkt der Harnsäure bei näherungsweise 254nm arbeitet. Da die Extinktionsdifferenz von Harnsäure an dem isosbestischen Punkt der Harnsäure Null ist, die von Kreatinin aber nicht, ist die Zuordnung der Extinktion zur Kreatininkonzentration möglich.

Eine Schar von Differenzspektren für Kreatinin und Harnsäure ist in Fig. 6 gezeigt. Dabei ist die mit 22 bezeichnete Kurvenschar ein Differenzspektrum von Kreatinin in mehreren Verdünnungsgraden und die mit 23 bezeichnete Kurvenschar ein Differenzspektrum von Harnsäure in mehreren Verdünnungsgraden. Zusätzlich ist ein Extinktionsspektrum 24 von Kreatinin bei pH-Wert 7,3 und ein Extinktionsspektrum 25 von Harnsäure bei pH-Wert 7,3 dargestellt.

Die Erfindung lässt sich wie folgt zusammenfassen. Es wird die Konzentration ausgewählter Stoffe in verschiedenen biologischen Flüssigkeiten durch die pHabhängige Änderung der Extinktion ermittelt. Bei den zu untersuchenden vorzugsweise biologischen Flüssigkeiten kann es sich um verschiedene Produkte des menschlichen Organismus handeln. Das Verfahren ist für Anwendungen in Blut, Plasma, Urin sowie frischer/verbrauchter Dialysierflüssigkeit gedacht, kann aber in anderen optisch klaren Lösungsmitteln zur Bestimmung absorbierender Stoffe genutzt werden. Insbesondere ist die Messung der Extinktionen von einer sich im Fluss befindlichen biologischen Flüssigkeit möglich, wobei die Messung während einer HD-Therapie stattfindet. Ferner ist es denkbar die Messungen nicht nur bei zwei unterschiedlichen pH-Werten durchzuführen sondern bei mehreren unterschiedlichen pH-Werten oder sogar kontinuierlich im Verlauf einer pH-Änderung.

Im Wesentlichen wird die Konzentration aus der Erfassung zweier Extinktionssignale bestimmt. Mit der Extinktion ist das dekadische Absorptionsmaß gemeint, welches in dieser Erfindung von einem optischen Sensor erfasst wird. Die Extinktionssignale werden zu verschiedenen Zeiten bei verschiedenen pH-Werten der biologischen Flüssigkeit aufgenommen und aus ihnen werden Konzentrationen des gewünschten Stoffes ermittelt.

Der pH-Wert der biologischen Flüssigkeit kann auf verschiedene Weise geändert werden, z.B. durch elektrolytische Verfahren oder durch die Zugabe von Säuren, Basen und Gasen wie CO₂. In der Dialyse kommen als Säuren saures Hämodialysekonzentrates und Zitronensäure in Frage, die durch eine entsprechende Vorrichtung in den Fluss zudispensiert werden und somit zur Änderung des pH-Wertes beitragen. Alternativ wird der pH-Wert in erfindungsgemäß bevorzugter Weise durch ein Mischungsverhältnis eingestellt. Das bedeutet, dass nur das Verhältnis zwischen der verbrauchten Dialysierflüssigkeit und der zugegebenen Säure bekannt sein muss, um den gewünschten pH-Wert einzustellen.

Mit dem optischen Sensor werden je nach Arbeitswellenlänge verschiedene Stoffe detektiert. Besonders gut eignet sich der isosbestische Punkt von Harnsäure bei 254nm dazu, Kreatinin optisch nachzuweisen, unabhängig von anderen vorhandenen interferierenden Stoffen. Ergänzend wird Harnsäure im Bereich um z.B. 300nm auf die gleiche Art und Weise bestimmt.

Die verbrauchte Dialysierflüssigkeit fließt in einem ersten Schritt durch den optischen Sensor, der ein erstes Extinktionssignal der Dialysierflüssigkeit erfasst. Danach fördert eine Pumpe aus einem Behälter saures Konzentrat in die verbrauchte Dialysierflüssigkeit und ändert damit den pH-Wert der Lösung. Zur besseren Durchmischung kann optional eine Mischstrecke vorgesehen sein. Die Ansteuerung der Pumpe wird vom Sensor selbst oder durch ein (nicht dargestelltes) Steuergerät übernommen. Der Sensor erfasst auch das zweite Extinktionssignal der Dialysierflüssigkeit. Aus den zwei Extinktionssignalen ermittelt der Sensor eine entsprechende Konzentration von Kreatinin und gibt diese aus.

Zum Ermitteln eines Stoffs in einer körperausgeschiedenen/entnommenen Flüssigkeit oder der verbrauchten Dialysierflüssigkeit und insbesondere zum Ermitteln eines Qualitätsmaßes für die Dialyse vorzugsweise der Konzentration ausgewählter Schadstoffe und/oder zum Ermitteln einer entfernten Gesamtmenge an urämischen Toxinen während einer Dialyse (Clearance bei der Dialyse) mit einer Dialysevorrichtung weist die erfindungsgemäße Analysevorrichtung sowie das eingesetzte Vorrichtungssteuerungverfahren folgendes auf:
Erfassung wenigstens eines ersten Extinktionssignals der körperausgeschiedenen/entnommenen Flüssigkeit oder der verbrauchten Dialysierflüssigkeit bei wenigstens einer vorgegebenen Analysewellenlänge bei einem definierten ggf. vorab einstellbaren ersten pH-Wert der Flüssigkeit durch einen Absorptionssensor (10),
Einstellung eines von dem ersten pH-Wert abweichenden zweiten pH-Wertes der Flüssigkeit durch eine pH-Einstelleinrichtung (9; 13; 15),
Erfassung wenigstens eines zweiten Extinktionssignals der Flüssigkeit bei der wenigstens einen vorgegebenen Analysewellenlänge beim zweiten pH-Wert durch den Absorptionssensor (10),
Verarbeitung/Vergleich der beiden Extinktionssignale für den ersten und zweiten pH-Wert sowie Ermittelung einer absoluten Konzentration des in der Flüssigkeit gelösten Stoffs.

### Bezugszeichen

- 1: Dialysator
- 2: Bluteingang
- 3: Blutausgang
- 4: Dialysierflüssigkeitseingang
- 5: Dialysierflüssigkeitsausgang
- 6: Dialysierflüssigkeitspumpe
- 7: Substanzbehälter mit pH-Wert-ändernder Substanz
- 8: Substanzpumpe
- 9: Mischstrecke
- 10: optischer Sensor
- 11: Verwurf, Entsorgung
- 12: Kommunikationsschnittstelle mit dem PC
- 13: Anpassungsbehälter zur Anpassung des pH-Wertes
- 14: Drei-Wege-Ventil
- 15: Elektrolysebehälter zur Anpassung des pH-Wertes mittels Elektrolyse
- 16: Extinktion unverbrauchte Dialysierflüssigkeit
- 17: Extinktion von Kreatinin bei verschiedenen Konzentrationen
- 18: Extinktion von Kreatinin bei pH-Wert -7.3
- 19: Extinktion von Kreatinin bei pH-Wert ∼4
- 20: lokales Maximum der Extinktion von Kreatinin bei pH-Wert 7,3
- 21: lokales Maximum der Extinktion von Kreatinin bei pH-Wert ∼4
- 22: Differenzspektrum von Kreatinin
- 23: Differenzspektrum von Harnsäure
- 24: Extinktionsspektrum von Kreatinin bei pH-Wert 7,3
- 25: Extinktionsspektrum von Harnsäure bei pH-Wert 7,3

## Patentansprüche

1. Extrakorporale Körperflüssigkeits-Reinigungsmaschine mit einer Konzentrationsbestimmungsvorrichtung zur Bestimmung quantitativer Mengen urämischer Toxine in einer Flüssigkeit, wobei die Flüssigkeit eine vom Körper ausgeschiedene/entnommene oder eine extrakorporale Blutreinigungsflüssigkeit ist, wobei
die Konzentrationsbestimmungsvorrichtung aufweist:
- einen optischen Absorptionssensor (10), der dafür angepasst ist, wenigstens ein erstes Extinktionssignal der körperausgeschiedenen/entnommenen Flüssigkeit oder verbrauchten Dialysierflüssigkeit bei wenigstens einer vorgegebenen oder einstellbaren Analysewellenlänge bei einem definierten oder einstellbaren ersten pH-Wert der körperausgeschiedenen/entnommenen Flüssigkeit oder verbrauchten Dialysierflüssigkeit zu erfassen,
- eine pH-Einstell-/Änderungseinrichtung (9; 13; 15), die dafür angepasst ist, einen von dem ersten pH-Wert abweichenden zweiten pH-Wert der körperausgeschiedenen/entnommenen Flüssigkeit oder verbrauchten Dialysierflüssigkeit einzustellen,
- den optischen Absorptionssensor (10), der dafür angepasst ist, wenigstens ein zweites Extinktionssignal der körperausgeschiedenen/entnommenen Flüssigkeit oder verbrauchten Dialysierflüssigkeit mit der wenigstens einen gleichen Analysewellenlänge bei dem zweiten pH-Wert zu erfassen,
- eine Signal-Verarbeitungseinrichtung, die dafür angepasst ist, die beiden Extinktionssignale bei dem ersten und dem zweiten pH-Wert zu verarbeiten, vorzugsweise zu vergleichen und
- eine Ermittlungseinrichtung, die dafür angepasst ist, eine absolute Konzentration oder ein Signal proportional zur Konzentration mindestens eines ausgewählten oder auswählbaren Stoffs, wie urämische Toxine, in der körperausgeschiedenen/entnommenen Flüssigkeit oder verbrauchten Dialysierflüssigkeit auf der Basis des Ergebnisses der Signal-Verarbeitungseinrichtung zu bestimmen.

2. Extrakorporale Körperflüssigkeits-Reinigungsmaschine mit einer Konzentrationsbestimmungsvorrichtung nach Anspruch 1 **gekennzeichnet durch** eine Analysewellenlängen-Einstelleinrichtung, die dafür angepasst ist, die Analysewellenlänge auf einen Wert einzustellen, bei welchem der ausgewählte oder auswählbare Stoff bei einer Änderung des pH Werts seine Absorptionseigenschaft ändert.

3. Extrakorporale Körperflüssigkeits-Reinigungsmaschine mit einer Konzentrationsbestimmungsvorrichtung nach Anspruch 2, **dadurch gekennzeichnet, dass** die Analysewellenlängen-Einstelleinrichtung dafür angepasst ist, die Analysewellenlänge auf einen Wert einzustellen, bei welchem ein nicht ausgewählter Stoff bei einer Änderung des pH Werts seine Absorptionseigenschaft nicht oder hinsichtlich der Signal-Verarbeitungseinrichtung vernachlässigbar ändert.

4. Extrakorporale Körperflüssigkeits-Reinigungsmaschine mit einer Konzentrationsbestimmungsvorrichtung nach einem der vorstehenden Ansprüche, **dadurch gekennzeichnet, dass** sie dafür angepasst ist, eine Dialysierflüssigkeit, Blut, Plasma, Urin oder dergleichen medizinische/biologische Lösungsmittel mittels des optischen Absorptionssensors zu analysieren.

5. Extrakorporale Körperflüssigkeits-Reinigungsmaschine mit einer Konzentrationsbestimmungsvorrichtung nach einem der vorstehenden Ansprüche, **dadurch gekennzeichnet, dass** der optische Absorptionssensor ein Ein-Wellenlängen-Sensor ist, der im Rahmen eines Analysevorgangs nur ein Signal mit einer einzigen vordefinierten oder auswählbaren Analysewellenlänge einsetzt.

6. Extrakorporale Körperflüssigkeits-Reinigungsmaschine mit einer Konzentrationsbestimmungsvorrichtung nach einem der vorstehenden Ansprüche, **dadurch gekennzeichnet, dass** die pH-Einstell-/Änderungseinrichtung (9; 13; 15) dafür angepasst ist, den ersten pH-Wert auf etwa 7.3 einzustellen.

7. Extrakorporale Körperflüssigkeits-Reinigungsmaschine mit einer Konzentrationsbestimmungsvorrichtung nach einem der vorstehenden Ansprüche, **dadurch gekennzeichnet, dass** die pH-Einstell-/Änderungseinrichtung (9; 13; 15) den pH-Wert der körperausgeschiedenen/entnommenen Flüssigkeit oder der Dialysierflüssigkeit verändert durch Anwendung eines elektrolytischen Verfahrens mittels einer Elektrolysezelle (13) oder durch Zugabe von Säuren, Basen und Gasen, wie CO₂.

8. Extrakorporale Körperflüssigkeits-Reinigungsmaschine mit einer Konzentrationsbestimmungsvorrichtung nach Anspruch 7, **dadurch gekennzeichnet, dass** die pH-Einstell-/Änderungseinrichtung (9; 13; 15) dafür angepasst ist, in der Ausbildung als Mischeinrichtung zumindest den ersten und/oder zweiten pH-Wert über das jeweilige Mischungsverhältnis zwischen der körperausgeschiedenen/entnommenen Flüssigkeit oder der Dialysierflüssigkeit und einem den pH-Wert verändernden Zugabestoff zu bestimmen.

9. Extrakorporale Körperflüssigkeits-Reinigungsmaschine mit einer Konzentrationsbestimmungsvorrichtung nach einem der vorstehenden Ansprüche, **dadurch gekennzeichnet, dass** im Fall von sich überlagernden Absorptionsbanden des ausgewählten Stoffs und zumindest eines weiteren, nicht ausgewählten Stoffs der Absorptionssensor bei einer isosbestischen Analysewellenlänge des nicht ausgewählten Stoffs arbeitet.

10. Extrakorporale Körperflüssigkeits-Reinigungsmaschine mit einer Konzentrationsbestimmungsvorrichtung nach Anspruch 9, bei der die Analysewellenlänge näherungsweise 254nm zur Analyse von Kreatinin beträgt, entsprechend dem isosbestischen Punkt von Harnsäure und/oder näherungsweise 300nm zur Analyse von Harnsäure beträgt.

11. Extrakorporale Körperflüssigkeits-Reinigungsmaschine mit einer Konzentrationsbestimmungsvorrichtung nach einem der vorstehenden Ansprüche, bei der eine Lichtquelle und/oder der Absorptionssensor in einem Wellenlängenbereich mit einer Breite von im wesentlichen +/- 1nm bis 5nm arbeitet.

12. Verfahren zur quantitativen Konzentrationsbestimmung urämischer Toxine in einer Flüssigkeit unter Verwendung einer extrakorporalen Körperflüssigkeits-Reinigungsmaschine gemäß einem der Ansprüche 1 bis 11, wobei die Flüssigkeit eine vom Körper ausgeschiedene/entnommene Flüssigkeit oder eine extrakorporale Blutreinigungsflüssigkeit ist sowie vorzugsweise zum Ermitteln eines Qualitätsmaßes für die Dialyse vorzugsweise der Konzentration ausgewählter Schadstoffe und/oder zum Ermitteln einer entfernten Gesamtmenge an urämischen Toxinen während einer Dialyse mit einer Dialysemaschine (1) wobei das Verfahren die Schritte aufweist:
- Erfassen wenigstens eines ersten Extinktionssignals einer vom Körper ausgeschiedenen/entnommenen Flüssigkeit oder einer verbrauchten Dialysierflüssigkeit bei wenigstens einer vorgegebenen oder ausgewählten Analysewellenlänge bei einem definierten oder einstellbaren ersten pH-Wert der körperausgeschiedenen / entnommenen Flüssigkeit oder der verbrauchten Dialysierflüssigkeit durch einen optischen Absorptionssensor (10),
- Einstellen eines von dem ersten pH-Wert abweichenden zweiten pH-Werts der körperausgeschiedenen/entnommenen Flüssigkeit oder der Dialysierflüssigkeit durch eine pH-Einstelleinrichtung (9; 13; 15),
- Erfassen wenigstens eines zweiten Extinktionssignals der körperausgeschiedenen/entnommenen Flüssigkeit oder der verbrauchten Dialysierflüssigkeit bei der wenigstens einen vorgegebenen oder ausgewählten Analysewellenlänge bei dem zweiten pH-Wert durch den Absorptionssensor (10),
- Verarbeiten der beiden Extinktionssignale bei dem ersten und dem zweiten pH-Wert vorzugsweise durch Differenzbildung und Ermitteln vorzugsweise durch Berechnen oder tabellarisches Bestimmen einer absoluten Konzentration oder eines Signals proportional zur Konzentration mindestens eines in der körperausgeschiedenen / entnommenen Flüssigkeit oder der verbrauchten Dialysierflüssigkeit gelösten ausgewählten Stoffs auf der Basis des Verarbeitungsergebnisses.

13. Verfahren nach Anspruch 12, bei dem im Fall sich überlagernder Absorptionsbanden des ausgewählten Stoffs und wenigstens eines weiteren, nicht ausgewählten Stoffs die wenigstens eine vorgegebene Analysewellenlänge eine isosbestische Analysewellenlänge bezüglich des nicht ausgewählten Stoffs ist

14. Verfahren nach Anspruch 13, bei dem die wenigstens eine vorgegebene Analysewellenlänge auf näherungsweise 254nm zur Analyse von Kreatinin eingestellt wird, entsprechend dem isosbestischen Punkt von Harnsäure und/oder auf näherungsweise 300nm eingestellt wird zur Analyse von Harnsäure.

## Claims

1. An extracorporeal body-fluid purification machine with a concentration determination apparatus for determining quantitative amounts of uremic toxins in a fluid, wherein the fluid is a fluid excreted/extracted from the body or an extracorporeal blood purification fluid, wherein
the concentration determination apparatus comprises:
- an optical absorption sensor (10) adapted to detect at least one first extinction signal of the fluid excreted/extracted from the body or the spent dialysate at at least one predetermined or adjustable analysis wavelength at a defined or adjustable first pH value of the fluid excreted/extracted from the body or the spent dialysate,
- a pH adjusting/changing device (9; 13; 15) adapted to adjust a second pH value of the fluid excreted/extracted from the body or the spent dialysate which differs from the first pH value,
- the optical absorption sensor (10) adapted to detect at least one second extinction signal of the fluid excreted/extracted from the body or the spent dialysate at the at least one equal analysis wavelength at the second pH value,
- a signal processing device adapted to process, preferably compare, the two extinction signals at the first and the second pH values, and
- a determination device adapted to determine an absolute concentration or a signal proportional to the concentration of at least one selected or selectable substance, such as uremic toxins, in the fluid excreted/extracted from the body or the spent dialysate on the basis of the result of the signal processing device.

2. The extracorporeal body-fluid purification machine with a concentration determination apparatus according to claim 1, **characterized by** an analysis wavelength adjusting device adapted to adjust the analysis wavelength to a value at which the selected or selectable substance changes its absorption property if the pH value changes.

3. The extracorporeal body-fluid purification machine with a concentration determination apparatus according to claim 2, **characterized in that** the analysis wavelength adjusting device is adapted to adjust the analysis wavelength to a value at which a non-selected substance does not change or negligibly changes its absorption property with respect to the signal processing device if the pH value is changed.

4. The extracorporeal body-fluid purification machine with a concentration determination apparatus according to one of the preceding claims, **characterized in that** it is adapted to analyze a dialysate, blood, plasma, urine or similar medical/biological solvents by means of the optical absorption sensor.

5. The extracorporeal body-fluid purification machine with a concentration determination apparatus according to one of the preceding claims, **characterized in that** the optical absorption sensor is a single wavelength sensor using only a signal with a single predefined or selectable analysis wavelength in the scope of an analysis process.

6. The extracorporeal body-fluid purification machine with a concentration determination apparatus according to one of the preceding claims, **characterized in that** the pH adjusting/changing device (9; 13; 15) is adapted to adjust the first pH value to approximately 7.3.

7. The extracorporeal body-fluid purification machine with a concentration determination apparatus according to one of the preceding claims, **characterized in that** the pH adjusting/changing device (9; 13; 15) changes the pH value of the fluid excreted/extracted from the body or the dialysate by using an electrolytic method by means of an electrolytic cell (13) or by adding acids, bases and gases, such as CO₂.

8. The extracorporeal body-fluid purification machine with a concentration determination apparatus according to claim 7, **characterized in that** the pH adjusting/changing device (9; 13; 15) in the design as a mixing device is adapted to determine at least the first and/or second pH value by means of the respective mixing ratio between the fluid excreted/extracted from the body or the dialysate and an additive changing the pH value.

9. The extracorporeal body-fluid purification machine with a concentration determination apparatus according to one of the preceding claims, **characterized in that,** in the case of overlapping absorption bands of the selected substance and at least one further, non-selected substance, the absorption sensor operates at an isosbestic analysis wavelength of the non-selected substance.

10. The extracorporeal body-fluid purification machine with a concentration determination apparatus according to claim 9, wherein the analysis wavelength is approximately 254 nm for the analysis of creatinine, corresponding to the isosbestic point of uric acid and/or approximately 300 nm for the analysis of uric acid.

11. The extracorporeal body-fluid purification machine with a concentration determination apparatus according to one of the preceding claims, wherein a light source and/or the absorption sensor operate in a wavelength range with a breadth of substantially +/- 1 nm to 5 nm.

12. A method for the quantitative concentration determination of uremic toxins in a fluid by using an extracorporeal body-fluid purification machine according to one of claims 1 to 11, wherein the fluid is a fluid excreted/extracted from the body or an extracorporeal blood purification fluid, and preferably for the determination of a quality measure for the dialysis, preferably the concentration of selected toxic substances, and/or for the determination of a removed total quantity of uremic toxins during the dialysis with a dialysis apparatus (1), wherein the method comprises the steps of:
- detecting, by an optical absorption sensor (10), at least one first extinction signal of a fluid excreted/extracted from the body or a spent dialysate at at least one predetermined or selected analysis wavelength at a defined or adjustable first pH value of the fluid excreted/extracted from the body or the spent dialysate,
- adjusting, by a pH adjusting device (9; 13; 15), a second pH value of the fluid excreted/extracted from the body or the dialysate which differs from the first pH value,
- detecting, by the absorption sensor (10), at least one second extinction signal of the fluid excreted/extracted from the body or the spent dialysate at the at least one predetermined or selected analysis wavelength at the second pH value,
- processing the two extinction signals at the first and the second pH values preferably by calculating the difference and determining, preferably by calculating or tabular determination, an absolute concentration or a signal proportional to the concentration of at least one selected substance dissolved in the fluid excreted/extracted from the body or the spent dialysate, on the basis of the result of processing.

13. The method according to claim 12, wherein in the case of overlapping absorption bands of the selected substance and at least one further, non-selected substance, the at least one predetermined analysis wavelength is an isosbestic analysis wavelength with respect to the non-selected substance.

14. The method according to claim 13, wherein the at least one predetermined analysis wavelength is adjusted to approximately 254 nm for the analysis of creatinine, corresponding to the isosbestic point of uric acid and/or approximately 300 nm for the analysis of uric acid.

## Revendications

1. Machine de purification de fluide corporel extracorporelle avec un dispositif de détermination de concentration pour déterminer des proportions quantitatives de toxine urémique dans un fluide, selon laquelle le fluide est un fluide de purification du sang éliminé/prélevé du corps ou extracorporel, selon lequel le dispositif de détermination de concentration présente :
- un capteur d'absorption optique (10) qui est adapté pour détecter au moins un premier signal d'extinction du fluide éliminé/prélevé du corps ou du fluide de dialyse consommé pour au moins une longueur d'onde d'analyse prédéfinie ou réglable pour une première valeur de pH définie ou réglable du fluide éliminé/prélevé du corps ou du fluide de dialyse consommé,
- un dispositif de réglage/modification de pH (9 ; 13 ; 15) qui est adapté pour régler une seconde valeur de pH différant de la première valeur de pH du fluide éliminé/prélevé du corps ou du fluide de dialyse consommé,
- le capteur d'absorption optique (10) qui est adapté pour détecter au moins un second signal d'extinction du fluide éliminé/prélevé du corps ou du fluide de dialyse consommé avec la au moins une même longueur d'onde d'analyse pour la seconde valeur de pH,
- un dispositif de traitement de signal qui est adapté pour traiter les deux signaux d'extinction pour la première et la seconde valeur de pH, de préférence pour les comparer et
- un dispositif de détermination qui est adapté pour déterminer une concentration absolue ou un signal proportionnel à la concentration d'au moins une substance sélectionnée ou sélectionnable, comme une toxine urémique, dans le fluide éliminé/prélevé du corps ou le fluide de dialyse consommé sur la base du résultat du dispositif de traitement de signal.

2. Machine de purification de fluide corporel extracorporelle avec un dispositif de détermination de concentration selon la revendication 1 **caractérisée par** un dispositif de réglage de longueur d'onde d'analyse qui est adapté pour régler la longueur d'onde d'analyse sur une valeur, selon laquelle la substance sélectionnée ou sélectionnable modifie sa propriété d'absorption lors d'une modification de la valeur du pH.

3. Machine de purification de fluide corporel extracorporelle avec un dispositif de détermination de concentration selon la revendication 2, **caractérisée en ce que** le dispositif de réglage de longueur d'onde d'analyse est adapté pour régler la longueur d'onde d'analyse sur une valeur, selon laquelle une substance non sélectionnée ne modifie pas sa propriété d'absorption lors d'une modification de la valeur du pH ou de manière négligeable en ce qui concerne le dispositif de traitement de signal.

4. Machine de purification de fluide corporel extracorporelle avec un dispositif de détermination de concentration selon l'une quelconque des revendications précédentes, **caractérisée en ce qu'**elle est adaptée pour analyser un fluide de dialyse, du sang, du plasma, de l'urine ou un diluant médical/biologique de ce genre au moyen du capteur d'absorption optique.

5. Machine de purification de fluide corporel extracorporelle avec un dispositif de détermination de concentration selon l'une quelconque des revendications précédentes, **caractérisée en ce que** le capteur d'absorption optique est un capteur à une longueur d'onde qui ne met en œuvre qu'un signal dans le cadre d'un processus d'analyse avec une seule longueur d'onde d'analyse prédéfinie ou sélectionnable.

6. Machine de purification de fluide corporel extracorporelle avec un dispositif de détermination de concentration selon l'une quelconque des revendications précédentes, **caractérisée en ce que** le dispositif de réglage/modification de pH (9 ; 13 ; 15) est adapté pour régler la première valeur de pH sur environ 7,3.

7. Machine de purification de fluide corporel extracorporelle avec un dispositif de détermination de concentration selon l'une quelconque des revendications précédentes, **caractérisée en ce que** le dispositif de réglage/modification de pH (9 ; 13 ; 15) modifie la valeur de pH du fluide éliminé/prélevé du corps ou du fluide de dialyse consommé en utilisant un procédé électrolytique au moyen d'une cellule d'électrolyse (13) ou par addition d'acides, de bases et de gaz, comme du CO₂.

8. Machine de purification de fluide corporel extracorporelle avec un dispositif de détermination de concentration selon la revendication 7, **caractérisée en ce que** le dispositif de réglage/modification de pH (9 ; 13 ; 15) est adapté, dans la configuration en tant que dispositif de mélange, pour déterminer au moins la première et/ou la seconde valeur de pH par l'intermédiaire du rapport de mélange respectif entre le fluide éliminé/prélevé du corps ou le fluide de dialyse consommé et un additif modifiant la valeur de pH.

9. Machine de purification de fluide corporel extracorporelle avec un dispositif de détermination de concentration selon l'une quelconque des revendications précédentes, **caractérisée en ce que**, en cas de superposition de bandes d'absorption de la substance sélectionnée et d'au moins une autre substance non sélectionnée, le capteur d'absorption fonctionne pour une longueur d'onde d'analyse isosbestique de la substance non sélectionnée.

10. Machine de purification de fluide corporel extracorporelle avec un dispositif de détermination de concentration selon la revendication 9, selon laquelle la longueur d'onde d'analyse est approximativement de 254 nm pour l'analyse de la créatinine, correspond au point isosbestique de l'acide urique et/ou est approximativement de 300 nm pour l'analyse de l'acide urique.

11. Machine de purification de fluide corporel extracorporelle avec un dispositif de détermination de concentration selon l'une quelconque des revendications précédentes, selon laquelle une source lumineuse et/ou le capteur d'absorption fonctionne dans une plage de longueurs d'onde d'une largeur essentiellement de +/-1 nm à 5 nm.

12. Procédé pour déterminer une concentration quantitative d'une toxine urémique dans un fluide en utilisant une machine de purification de fluide corporel extracorporelle selon l'une des revendications 1 à 11, selon lequel le fluide est un fluide éliminé/prélevé du corps ou un fluide de purification du sang extracorporel, ainsi que, de préférence, pour déterminer une mesure de qualité pour la dialyse, de préférence la concentration de substances nocives sélectionnées et/ou pour déterminer une quantité totale éliminée de toxines urémiques pendant une dialyse avec une machine de dialyse (1), selon lequel le procédé présente les étapes :
- de détection d'au moins un premier signal d'extinction d'un fluide éliminé/prélevé du corps ou d'un fluide de dialyse consommé pour au moins une longueur d'onde d'analyse prédéfinie ou sélectionnée pour une première valeur de pH définie ou réglable du fluide éliminé/prélevé du corps ou du fluide de dialyse consommé par un capteur d'absorption optique (10),
- de réglage d'une seconde valeur de pH différant de la première valeur de pH du fluide éliminé/prélevé du corps ou du fluide de dialyse par un dispositif de réglage de pH (9 ; 13 ; 15),
- de détection d'au moins un second signal d'extinction du fluide éliminé/prélevé du corps ou du fluide de dialyse consommé pour la au moins une longueur d'onde d'analyse prédéfinie ou sélectionnée pour la seconde valeur de pH par le capteur d'absorption (10),
- de traitement des deux signaux d'extinction pour la première et la seconde valeur de pH de préférence par la formation d'une différence et de détermination de préférence en calculant ou en déterminant sous forme de table une concentration absolue ou un signal proportionnel à la concentration d'au moins une substance sélectionnée dissoute dans le fluide éliminé/prélevé du corps ou le fluide de dialyse consommé sur la base du résultat du traitement.

13. Procédé selon la revendication 12, selon lequel, en cas de superposition de bandes d'absorption de la substance sélectionnée et d'au moins une autre substance non sélectionnée, la au moins une longueur d'onde d'analyse prédéfinie est une longueur d'onde d'analyse isosbestique relative à la substance non sélectionnée.

14. Procédé selon la revendication 13, selon lequel la au moins une longueur d'onde d'analyse prédéfinie est réglée sur approximativement 254 nm pour l'analyse de la créatinine, est réglée sur une valeur correspondant au point isosbestique de l'acide urique et/ou sur approximativement 300 nm pour l'analyse de l'acide urique.
